# EUROPEAN PATENT APPLICATION

(11) **EP 1 223 171 A1**
(43) Date of publication of application: **17.07.2002**
(21) Application number: 02250043.3
(22) Date of filing: 04.01.2002
(51) Int. Cl.: C07D 417/12, C07D 275/02, A01N 43/80

(54) **Antimicrobial 5-substituted-3-isothiazolone compounds and methods of use**

(30) Priority: 16.01.2001 US 261725 P
(71) Applicant: Rohm and Haas, Philadelphia, Pennsylvania 19106-2399 (US)
(72) Inventor: Ghosh, Tirthankar, Oreland, Pennsylvania 19075 (US); Lange, Barry Clifford, Lansdale, Pennsylvania 19446 (US); Warwick, Eileen Fleck, Lansdale, Pennsylvania 19446 (US)
(74) Representative: Kent, Venetia Katherine

(57) **Abstract**

Novel antimicrobial compounds based on 5-substituted 3-isothiazolones, having Formula I: 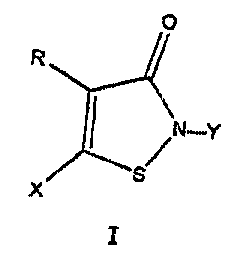 where the 5-substituent is an aryl or heterocyclic ether/thioether group, are disclosed. These 5-substituted 3-isothiazolones provide enhanced antimicrobial activity over that of related 3-isothiazolones, particularly when the 5-substituent is a substituted pyridinyl thioether group.

## Description

### BACKGROUND

This invention relates to novel compounds having antimicrobial activity and their use for controlling the growth of microogranisms, such as bacteria, fungi, algae and yeasts. In particular the invention involves 3-isothiazolones substituted at the 5-position with selected ether or thioether substituents.

U.S. Patent Nos. 3,546,235 and 3,712,908 disclose the reaction of 5-chloro-1,2-dithiol-3-one with metal salts of thiols by nucleophilic substitution to give the corresponding sulfides, unaccompanied by ring-opening; for example, 4,5-dichloro-1,2-dithiol-3-one reacted with sodium 2-mercaptobenzothiazole to provide the corresponding thioether. U.S. Patent No. 5,091,399 disclosed the reaction of 5-chloro-3-isothiazolone derivatives with sodium 2-mercaptobenzothiazole to provide the thioether analog of the 3-isothiazolone compound.

Although certain 5-substituted 3-isothiazolones are known antimicrobials, they provide a varying range of efficacy depending on the end use conditions. The problem addressed by the present invention is to provide new antimicrobial compounds that offer enhanced efficiency over that of related antimicrobial compounds.

### STATEMENT OF INVENTION

The present invention provides compounds having Formula **I**: 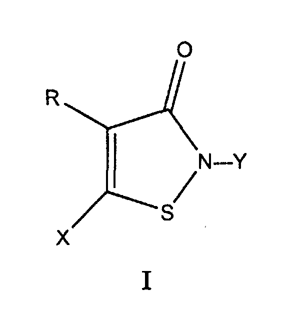 wherein Y is an unsubstituted or substituted (C₁-C₁₈)alkyl group, an unsubstituted or substituted (C₃-C₁₈)alkenyl or alkynyl group, an unsubstituted or substituted (C₅-C₁₂)cycloalkyl group, an unsubstituted or substituted (C₇-C₁₀)aralkyl group, or an unsubstituted or substituted (C₆-C₁₀)aryl group; R is a hydrogen, halogen or (C₁-C₄)alkyl group; and X is R₁S- or R₁O- where R₁ is selected from aryl and heterocyclic groups, provided that when R₁ is a heterocyclic group it is not a benzothiazolyl group.

In a preferred embodiment, the compound of Formula **I** is selected from [2-methyl-3-isothiazolon-5-yl]-(1-oxypyridin-2-yl)sulfide, [4-chloro-2-(n-octyl)-3-isothiazolon-5-yl]-(1-oxypyridin-2-yl)sulfide, [4-methyl-2-(4-chlorophenyl)-3-iso-thiazolon-5-yl]-(1-oxypyridin-2-yl)sulfide, [4-chloro-2-cyclohexyl-3-isothiazolon-5-yl]-(1-oxypyridin-2-yl)sulfide, [4-chloro-2-benzyl-3-isothiazolon-5-yl]-(1-oxypyridin-2-yl)sulfide, [2-(4-chlorophenyl)-3-isothiazolon-5-yl]-(1-oxypyridin-2-yl)sulfide, [2-(4-chlorobenzyl)-5-chloro-3-isothiazolon-5-yl]-(1-oxypyridin-2-yl)sulfide and [2-(4-chlorophenethyl)-5-chloro-3-isothiazolon-5-yl]-(1-oxypyridin-2-yl)sulfide.

In another aspect the present invention provides a method for controlling the growth of bacteria, fungi, algae and yeasts comprising introducing a microbicidally effective amount of a compound having Formula **I** to a locus that is subject to microbial attack, wherein the locus is selected from cooling towers, mineral slurries, pulp and paper processing fluids, plastics, emulsions, dispersions, paints, latexes, coatings, construction products, marine structures, household products, cosmetics, toiletries, shampoos, soaps, detergents, industrial cleaners, metalworking fluids, textiles and textile products, wood and wood products, surfactants and diagnostic reagents.

### DETAILED DESCRIPTION

We have discovered that certain 5-substituted 3-isothiazolone compounds provide enhanced antimicrobial activity and that they may be prepared by reacting selected oxygen or sulfur nucleophilic agents with 5-substituted-3-isothiazolone reactants.

As used herein, the following terms have the designated definitions, unless the context clearly indicates otherwise. The term "microbicide" is considered equivalent to "antimicrobial" as used herein and refers to a compound capable of inhibiting the growth of or controlling the growth of microorganisms at a locus; microbicides include bactericides, fungicides and algaecides. The term "microorganism" includes, for example, fungi, yeast, bacteria and algae. The term "locus" refers to an industrial system or product subject to contamination by microorganisms. All percentages referred to will be expressed in weight percent (%), based on total weight of composition involved, unless specified otherwise. The following abbreviations are used herein: g = grams; ml = milliliter, ppm = parts per million by weight/volume. Unless otherwise specified, ranges listed are to be read as inclusive and combinable and temperatures are in degrees centigrade (°C).

As used herein, the term "substituted alkyl group" refers to an alkyl group having one or more of its hydrogens replaced by another substituent group; examples of a substituted alkyl group include hydroxyalkyl, haloalkyl and alkylamino. By a "substituted aralkyl group" is meant an aralkyl group having one or more of its hydrogens on either the aryl ring or the alkyl chain replaced by another substituent group, including, for example, halo, nitro, carboxy, cyano, (C₁-C₄)alkyl, halo-(C₁-C₄)-alkoxy, (C₁-C₄)alkoxy and haloalkyl substituent groups.

As used herein, the term "aryl" refers to phenyl or naphthyl which may be optionally substituted ("substituted aryl group"). If the aryl group is substituted there may be up to three substituents independently selected from (C₁-C₄)alkyl, aryl, halo, carboxy, cyano, nitro, (C₁-C₄)alkoxy, halo-(C₁-C₄)alkoxy and haloalkyl. Suitable aryl (and substituted aryl) groups include, for example, phenyl, 4-chlorophenyl, 4-fluorophenyl, 4-bromophenyl, 3-nitrophenyl, 2-methoxyphenyl, 2-methylphenyl, 3-methylphenyl, 4-methylphenyl, 4-ethylphenyl, 2-methyl-3-methoxyphenyl, 2,4-dibromophenyl, 3,5-difluorophenyl, 3,5-dimethylphenyl, 2,4,6-trichlorophenyl, 4-methoxyphenyl, 4-(trifluoromethoxy)phenyl, naphthyl, 2-chloronaphthyl, 2,4-dimethoxyphenyl, 4-(trifluoromethyl)phenyl, 2-iodo-4-methylphenyl, 2-carboxyphenyl, 4-carboxyphenyl, 2-nitrophenyl and 4-nitrophenyl.

As used herein, the term "heterocyclic" refers to (i) a substituted or unsubstituted, saturated or unsaturated, 5 or 6 membered ring containing one, two, three or four heteroatoms (preferably one or two heteroatoms) independently selected from oxygen, nitrogen and sulfur; (ii) a substituted or unsubstituted bicyclic ring system containing up to 10 atoms including at least one heteroatom selected from oxygen, nitrogen and sulfur. If the heterocyclic ring is substituted there may be up to three substituents independently selected from (C₁-C₄)alkyl, aryl, heterocyclic, halogen, carboxy, cyano, nitro, (C₁-C₄)-alkoxy, halo-(C₁-C₄)-alkoxy and haloalkyl.

Suitable heterocyclic groups include, for example, tetrahydrofuryl, pyrrolidinyl, piperidinyl, tetrahydropyranyl, morpholinyl, piperazinyl, dioxolanyl, dioxanyl, indolinyl, 5-methyl-6-chromanyl, pyridinyl (2-, 3- or 4-isomers), 1-oxypyridinyl (2-, 3- or 4- isomers), pyrazinyl, pyrimidinyl (2-, 4- or 5-isomers), pyridazinyl, pyrazolyl, triazolyl, tetrazolyl, imidazolyl, thienyl (2- or 3-isomers), furyl (2- or 3- isomers), purinyl, pyrrolyl, oxazolyl, isoxazolyl, thiazolyl, isothiazolyl, oxadiazolyl, thiadiazolyl, quinazolyl, quinazolonyl, quinolyl, isoquinolyl, benzofuranyl and benzothiofuranyl (benzothienyl). Preferably, the heterocyclic group is selected from 2-pyridinyl, 3-pyridinyl, 4-pyridinyl, 1-oxy-pyridin-2-yl, 1-oxypyridin-3-yl and 1-oxypyridin-4-yl.

Preferred compounds of Formula **I** are those where Y is selected from methyl, n-octyl, cyclohexyl and 4-chlorophenyl; more preferably, the compounds are [2-methyl-3-isothiazolon-5-yl]-(1-oxypyridin-2-yl)sulfide, [4-chloro-2-(n-octyl)-3-isothiazolon-5-yl]-(1-oxypyridin-2-yl)sulfide and [4-methyl-2-(4-chlorophenyl)-3-isothiazolon-5-yl]-(1-oxypyridin-2-yl)sulfide. As described herein, the aforementioned "sulfide" designations will be used to name the sulfide compounds of Formula **I**. However, according to IUPAC (International Union of Pure and Applied Chemistry) nomenclature these sulfide compounds would also be referred to as "sulfanyl" derivatives of the parent 3-isothiazolone compound, for example, compound **3** (see Scheme **A)** would be designated 2-methyl-5-(1-oxypyridin-2-yl sulfanyl)-3-isothiazolone.

The compounds of Formula **I** may be prepared according to Scheme **A**. In general, a 5-substituted 3-isothiazolone (represented by compound **1**) is allowed to undergo nucleophilic substitution at the 5-position with the salt form of an oxygen-containing (for example, substituted or unsubstituted phenoxide) or sulfur-containing reactant (for example, sulfide such as compound **2**). The 5-substituted 3-isothiazolone reactant may be any 3-isothiazolone with an acceptable leaving group in the 5-position; however, 5-halo-3-isothiazolones are preferred and the 5-chloro derivatives are more preferred. The nucleophilic oxygen-containing or sulfur-containing reactant is typically the salt form (for example sodium, potassium, lithium or zinc salt) of a phenolic, thiophenolic or mercapto-substituted heterocyclic compound. Suitable phenolic compounds useful as reactants to provide the antimicrobial compounds of the present invention include, for example, 4-nitrophenol, 2,4-dinitrophenol, 4-chlorophenol, 2,4-dichlorophenol, 1-naphthol and 2-naphthol. Suitable mercapto-substituted heterocyclic and thiophenolic compounds useful as reactants to provide compounds of the present invention include, for example, 2-mercaptopyridine-N-oxide, 2-mercaptopyridine, 4-mercaptopyridine, 2-mercaptopyrimidine, 2-mercapto-4(3H)-quinazolinone, 6-mercaptopurine, 4-mercapto-1H-pyrazolo-[3,4-d]-pyrimidine, 5-mercapto-1-phenyltetrazole, 5-mercapto-2-nitrobenzoic acid, 4-nitrothiophenol and 4-mercaptobenzoic acid.

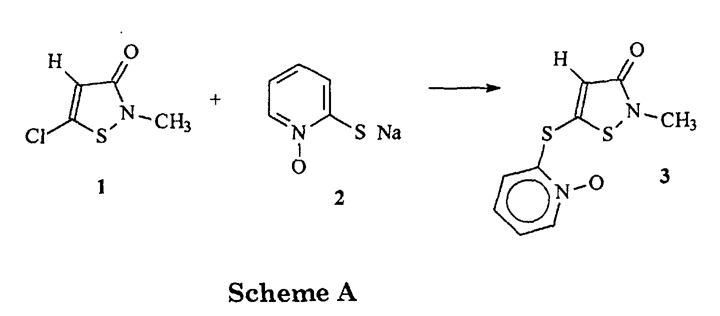

For example, the 5-halo-3-isothiazolone derivative (compound 1, 5-chloro-2-methyl-3-isothiazolone in Scheme **A)** is contacted with the sodium salt of 2-mercaptopyridine-N-oxide (compound **2**) in aqueous ethanol solution to give compounds of Formula **I** (compound **3**, corresponding to Formula **I** where Y = methyl, R = hydrogen, X = R₁S- and R₁ = 1-oxypyridin-2-yl). The reaction takes place at room temperature, under alkaline condition (pH = 8 to 10) and is complete within 1 to 96 hours.

The compounds of Formula **I** may also be prepared in an anhydrous organic solvent as follows: 2-mercaptopyridine-N-oxide is treated with sodium hydride in an anhydrous solvent such as dimethyl formamide or acetonitrile at 0°C under nitrogen to produce compound **2.** A solution of 5-chloro-3-isothiazolone derivative (such as compound **1)** in the same solvent is then added. The mixture is allowed to warm to room temperature and then stirred for 1 to 24 hours to complete the reaction. The product (such as compound **3**) typically precipitates out of solution or is isolated by extraction.

The compounds of Formula **I** may also be prepared *in-situ*, that is, the individual reactants (5-halo-3-isothiazolone and oxygen or sulfur nucleophilic agent) may be added separately into an end-use environment and allowed to react to form the desired compounds of Formula **I**.

The antimicrobial compounds of the present invention can be used to inhibit or control the growth of microorganisms (bacteria, fungi, algae and yeasts) by introducing a microbicidally effective amount of the compound to a locus that is subject to microbial attack. The amount of compound to be used depends on the application. Typically the amount of compound of Formula **I** incorporated into a locus is from 0.1 to 10,000 ppm, preferably from 0.5 to 5,000 ppm and more preferably from 1 to 1000 ppm.

Suitable loci include, for example: cooling towers; air washers; boilers; mineral slurries; wastewater treatment; ornamental fountains; marine structures, such as boats, ships, oil platforms, piers, pilings, docks, elastomeric rubbers and fish nets; marine antifouling coatings, such as marine paints and varnishes; reverse osmosis filtration; ultrafiltration; ballast water; evaporative condensers; heat exchangers; pulp and paper processing fluids; plastics; emulsions and dispersions; paints; latexes; coatings, such as varnishes; construction products, such as mastics, caulks, and sealants; construction adhesives, such as ceramic adhesives, carpet backing adhesives, and laminating adhesives; industrial or consumer adhesives; photographic chemicals; printing fluids; household products, such as bathroom disinfectants or sanitizers; cosmetics and toiletries; shampoos; soaps; detergents; industrial disinfectants or sanitizers, such as cold sterilants, hard surface disinfectants; floor polishes; laundry rinse water; metalworking fluids; conveyor lubricants; hydraulic fluids; leather and leather products; textiles; textile products; wood and wood products, such as plywood, chipboard, flakeboard, laminated beams, oriented strandboard, hardboard, and particleboard; petroleum processing fluids; fuel; oilfield fluids, such as injection water, fracture fluids, and drilling muds; agriculture adjuvant preservation; surfactant preservation; medical devices; diagnostic reagent preservation; food preservation, such as plastic or paper food wrap; and pools and spas.

Preferably, the antimicrobial compounds of the present invention are used to inhibit the growth of microorganisms at a locus selected from cooling towers, mineral slurries, pulp and paper processing fluids, plastics, emulsions, dispersions, paints, latexes, coatings, construction products, marine structures, household products, cosmetics, toiletries, shampoos, soaps, detergents, industrial cleaners, metalworking fluids, textiles and textile products, wood and wood products, surfactants and diagnostic reagents. In particular, microbicidal compositions containing compounds of the present invention are useful in personal care applications, such as hair care (for example, shampoo and dyes), skin care (for example, sunscreens, cosmetics, soaps, lotions and creams), and marine antifoulant (for example, marine paints and varnishes) formulations.

Optionally, the compounds of Formula **I** can be used in combination with other microbicidal compounds. Formulated compositions comprising an effective amount of a compound of Formula **I** with an acceptable carrier may be used or the compounds may be applied directly to the end-use environment.

Some embodiments of the invention are described in detail in the following Examples. All ratios, parts and percentages are expressed by weight unless otherwise specified, and all reagents used are of good commercial quality unless otherwise specified.

### Example 1

### Preparation of Compound 3: [2-methyl-3-isothiazolon-5-yl]-(1-oxypyridin-2-yl)sulfide (see Scheme A):

To a well stirred solution of 0.5 g (0.003 mol) of 5-chloro-2-methyl-3-isothiazolone (compound **1**) in 9.6 g ethanol was added 1.0 g (0.003 mol) of a 40% aqueous solution of 2-mercaptopyridine-N-oxide, sodium salt (compound **2**). The pH of the solution was adjusted to 8.5 by using a 0.5% sodium hydroxide solution (aqueous). The reaction mixture was stirred for an additional 4 hours and then the ethanol was removed under reduced pressure. A yellow solid was obtained which was washed with ethyl acetate. The solid was dissolved in chloroform and the solution was filtered. Removal of the chloroform from the filtrate, under reduced pressure, gave 0.45 g (70%) of compound **3** as a yellow solid: mp 141-143°C; ¹H NMR (CDCl₃, 300 MHz), δ 3.30 (s, 3H), 6.50 (s, 1H), 7.20 (m, 3H), 8.20 (m, 1H); ¹³C NMR (CDCl₃), δ 31.1, 124.0, 124.2, 124.7, 127.3, 139.2, 145.0, 148.9, 168.4. Elemental analysis: calculated for C₉H₈N₂O₂S₂: C, 45.0; H, 3.4; N, 11.7; found: C, 44.9; H, 3.4; N, 11.4.

### Example 2

### Preparation of Compound 5: [4-chloro-2-n-octyl-3-isothiazolon-5-yl]-(1-oxypyridin-2-yl)sulfide (see Scheme B):

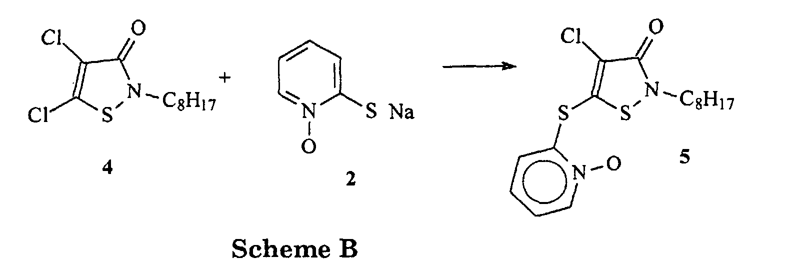

To a well stirred solution of 1.2 g (0.004 mol) of 4,5-dichloro-2-n-octyl-3-isothiazolone (compound **4**) in 8.8 g of ethanol was added 1.1 g (0.003 mol) of a 40% aqueous solution of 2-mercaptopyridine-N-oxide, sodium salt (compound **2**). The pH of the solution was adjusted to 8.5 by using a 0.5% sodium hydroxide solution (aqueous). The reaction mixture was stirred for an additional 4 hours and then the ethanol was removed under reduced pressure. The sticky solid obtained was dissolved in methylene chloride and washed repeatedly with water. The methylene chloride was dried over anhydrous magnesium sulfate and the methylene chloride removed under reduced pressure to give 0.9 g (82%) of compound **5** as a yellow oil: ¹H NMR (CDCl₃, 300 MHz), δ 0.80 (m, 3H), 1.23 (m, 10H), 1.70 (m, 2H), 3.80 (m, 2H), 7.01 (m, 1H), 7.22 (m, 2H), 8.25 (m, 1H); ¹³C NMR (CDCl₃), δ 13.1, 21.6, 25.5, 25.9, 28.0, 28.4, 30.7, 44.6, 122.1, 122.5, 122.8, 125.8, 135.5, 137.8, 145.4, 161.4. Elemental analysis: calculated for C₁₆H₂₁ClN₂O₂S₂: C, 51.5; H, 5.7; N, 7.5; found: C, 51.4, H, 5.8, N, 7.3.

### Example 3 (comparative)

### Preparation of Compound 6: [2-methyl-3-isothiazolon-5-yl]-(benzothiazol-2'-yl)sulfide.

In a manner similar to that described in Example 1, compound **6** was prepared using the sodium salt of 2-mercaptobenzothiazole in place of compound **2**. The resulting compound **6** was recrystallized from ethanol; m.p. 143-145°C. U.S. Patent No. 5,091,399 may be consulted for further general and specific details on preparation of this benzothiazolyl derivative.

### Example 4

Biological Activity: certain compounds of the present invention were tested for activity against three microorganisms, *Escherichia coli* 8739 (Gram-negative bacterium) and *Staphylococcus aureus* 6538 (Gram-positive bacterium) and *Aspergillus niger* 16404.

The lowest concentration of test compound required to inhibit *Staphylococcus aureus* 6538, *Escherichia coli* 8739 and *Aspergillus niger* 16404 was determined by a high resolution minimum inhibitory concentration (HRMIC) test. Varying amounts of the test compounds were added to minimal salts glucose medium (see T. Maniatis, E.F. Fritsch and J. Sambrook, Molecular Cloning, p 68 (1982)) supplemented with 0.1% yeast extract (M9GY) in a 96-well microtiter plate. Ten-fold serial dilutions were performed on a Biomek™ 2000 Workstation to obtain a range of closely spaced concentrations of test compound as shown in Table 1 (based on dilutions of a 10,000 ppm active ingredient stock solution of the test compound). The plates were inoculated with cell suspension of inoculum and adjusted to provide about 10⁶ CFU (colony forming units)/ml in each well. The microtiter plates were incubated at 30°C for 24 hours and then checked (optical density at 650 nm using a microplate reader) for the presence or absence of microbial growth in each well. The concentration of compound in the first microtiter well demonstrating "no growth" was designated as the minimum inhibitory concentration (MIC) for the test compound. Data reported (ppm active ingredient) are the average of multiple determinations (see Table 2).

**Table 1**

| Concentration Profile (active ingredient) in HRMIC Test | | | | | | |
|---|---|---|---|---|---|---|
| | **Test Compound Concentration (ppm)** | | | | | |
| | **1** | **2** | **3** | **4** | **5** | **6** |
| **A** | 1000 | 100 | 10 | 1 | 0.1 | 0 |
| **B** | 800 | 80 | 8 | 0.8 | 0.08 | 0 |
| **C** | 700 | 70 | 7 | 0.7 | 0.07 | 0 |
| **D** | 600 | 60 | 6 | 0.6 | 0.06 | 0 |
| **E** | 500 | 50 | 5 | 0.5 | 0.05 | 0 |
| **F** | 400 | 40 | 4 | 0.4 | 0.04 | 0 |
| **G** | 300 | 30 | 3 | 0.3 | 0.03 | 0 |
| **H** | 200 | 20 | 2 | 0.2 | 0 | 0 |

The compounds of the present invention offer effective control of both bacterial and fungal microorganisms compared to isothiazolonyl-benzothiazolyl sulfide derivatives of the prior art (compound **6**). Compounds **3** and **5** of the present invention (Table 2) provide 5-fold and at least 30- to 160-fold better control, respectively, versus Gram-positive (*S*. *aur)* and Gram-negative *(E. coli*) bacteria, respectively, than compound **6.** While providing enhanced control of both Gram-positive and Gram-negative bacteria relative to compound **6,** compounds **3** and **5** also provide effective control of fungal organisms (*A*. *niger).*

**Table 2**

| Microbicidal Efficacy of Compounds of Formula **I** | | | |
|---|---|---|---|
| **Compound** | **MIC (ppm)** | | |
| | ***S*. *aur**** | ***E*. *coli**** | ***A*. *niger**** |
| **3** | 1 | 6 | 60 |
| **5** | 0.9 | 30 | 10 |
| **6** (comparative) | 5 | >1000 | 15 |
| *S. aur = Staphylococcus aureus | | | |
| E. coli = Escherichia coli | | | |
| A. niger = Aspergillus niger | | | |

## Claims

1. A compound having Formula **I**: 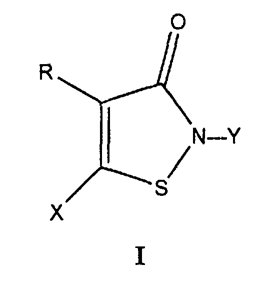 wherein:
Y is an unsubstituted or substituted (C₁-C₁₈)alkyl group, an unsubstituted or substituted (C₃-C₁₈)alkenyl or alkynyl group, an unsubstituted or substituted (C₅-C₁₂)cycloalkyl group, an unsubstituted or substituted (C₇-C₁₀)aralkyl group, or an unsubstituted or substituted (C₆-C₁₀)aryl group;
R is a hydrogen, halogen or (C₁-C₄)alkyl group; and
X is R₁S- or R₁O- where R₁ is selected from aryl and heterocyclic groups, provided that when R₁ is a heterocyclic group it is not a benzothiazolyl group.

2. The compound of claim 1 wherein Y is selected from methyl, n-octyl, cyclohexyl and 4-chlorophenyl.

3. The compound of claim 1 wherein when R₁ is an aryl group, R₁ is selected from phenyl, 4-chlorophenyl, 4-fluorophenyl, 4-bromophenyl, 3-nitrophenyl, 2-methoxyphenyl, 2-methylphenyl, 3-methylphenyl, 4-methylphenyl, 4-ethylphenyl, 2-methyl-3-methoxyphenyl, 2,4-dibromophenyl, 3,5-difluorophenyl, 3,5-dimethylphenyl, 2,4,6-trichlorophenyl, 4-methoxyphenyl, 4-(trifluoro-methoxy)phenyl, naphthyl, 2-chloronaphthyl, 2,4-dimethoxyphenyl, 4-(trifluoro-methyl)phenyl, 2-iodo-4-methylphenyl, 2-carboxyphenyl, 4-carboxyphenyl, 2-nitrophenyl and 4-nitrophenyl.

4. The compound of claim 1 wherein when R₁ is a heterocyclic group, R₁ is selected from tetrahydrofuryl, pyrrolidinyl, piperidinyl, tetrahydropyranyl, morpholinyl, piperazinyl, dioxolanyl, dioxanyl, indolinyl, 5-methyl-6-chromanyl, 2-pyridinyl, 3-pyridinyl, 4-pyridinyl, 1-oxypyridin-2-yl, 1-oxypyridin-3-yl, 1-oxy-pyridin-4-yl, pyrazinyl, 2-pyrimidinyl, 4-pyrimidinyl, 5-pyrimidinyl, pyridazinyl, pyrazolyl, triazolyl, tetrazolyl, imidazolyl, 2-thienyl, 3-thienyl, 2-furyl, 3-furyl, purinyl, pyrrolyl, oxazolyl, isoxazolyl, thiazolyl, isothiazolyl, oxadiazolyl, thiadiazolyl, quinazolyl, quinazolonyl, quinolyl, isoquinolyl, benzofuranyl and benzothiofuranyl.

5. The compound of claim 1 wherein R₁ is selected from 2-pyridinyl, 3-pyridinyl, 4-pyridinyl, 1-oxypyridin-2-yl, 1-oxypyridin-3-yl and 1-oxypyridin-4-yl.

6. The compound of claim 1 wherein the compound is selected from [2-methyl-3-isothiazolon-5-yl]-(1-oxypyridin-2-yl)sulfide, [4-chloro-2-(n-octyl)-3-isothiazolon-5-yl]-(1-oxypyridin-2-yl)sulfide, [4-methyl-2-(4-chlorophenyl)-3-iso-thiazolon-5-yl]-(1-oxypyridin-2-yl)sulfide, [4-chloro-2-cyclohexyl-3-isothiazolon-5-yl]-(1-oxypyridin-2-yl)sulfide, [4-chloro-2-benzyl-3-isothiazolon-5-yl]-(1-oxypyridin-2-yl)sulfide, [2-(4-chlorophenyl)-3-isothiazolon-5-yl]-(1-oxypyridin-2-yl)sulfide, [2-(4-chlorobenzyl)-5-chloro-3-isothiazolon-5-yl]-(1-oxypyridin-2-yl)sulfide and [2-(4-chlorophenethyl)-5-chloro-3-isothiazolon-5-yl]-(1-oxypyridin-2-yl)sulfide.

7. A method for controlling the growth of bacteria, fungi, algae and yeasts comprising introducing a microbicidally effective amount of a compound of claim 1 to a locus that is subject to microbial attack.

8. The method of claim 7 wherein the locus is selected from cooling towers, mineral slurries, pulp and paper processing fluids, plastics, emulsions, dispersions, paints, latexes, coatings, construction products, marine structures, household products, cosmetics, toiletries, shampoos, soaps, detergents, industrial cleaners, metalworking fluids, textiles and textile products, wood and wood products, surfactants and diagnostic reagents.
